# EUROPEAN PATENT SPECIFICATION

(11) **EP 4 247 306 B1**
(45) Date of publication and mention of the grant of the patent: **04.11.2026**
(21) Application number: 21895881.7
(22) Date of filing: 18.11.2021
(51) Int. Cl.: A61F 2/848, A61F 2/90

(54) **FUSIFORM ANEURYSM TREATMENT**
BEHANDLUNG FUSIFORMER ANEURYSMEN
TRAITEMENT D'ANÉVRISME FUSIFORME

(30) Priority: 18.11.2020 US 202063115486 P
(43) Date of publication of application: 27.09.2023
(73) Proprietor: Microvention, Inc., Aliso Viejo, California 92656 (US)
(72) Inventor: RANGWALA, Hussain, Aliso Viejo, California 92656 (US); LIN, Tao, Aliso Viejo, California 92656 (US)
(74) Representative: KIPA AB
(86) International application number: PCT/US2021/072491
(87) International publication number: WO 2022/109584

(56) References cited:
- CN-U- 210 056 359
- JP-B2- 5 749 544
- US-A- 5 575 818
- US-A1- 2005 222 669
- US-A1- 2010 274 345
- US-A1- 2013 289 690
- US-A1- 2014 222 144
- US-A1- 2014 379 010
- US-A1- 2016 030 155
- US-A1- 2016 045 201
- US-A1- 2018 021 045
- US-A1- 2019 269 498
- US-A1- 2019 269 533
- US-A1- 2020 069 412
- US-A1- 2020 214 867
- US-B2- 8 500 792

## Description

### RELATED APPLICATIONS

This application claims benefit of and priority to U.S. Provisional Application Serial No. 63/115,486 filed November 18, 2020 entitled *Fusiform Aneurysm Treatment,* which is hereby referred to.

### BACKGROUND OF THE INVENTION

Aneurysms typically involve a bulging or deformation of a region of a blood vessel. This bulging can occur for a number of reasons, including weakening of the vessel wall and high pulsatile blood flow against a region of the vessel. Over time, the cavity can increase in size as blood continues to flow into it, increasing the risk of rupture or hemorrhagic stroke.

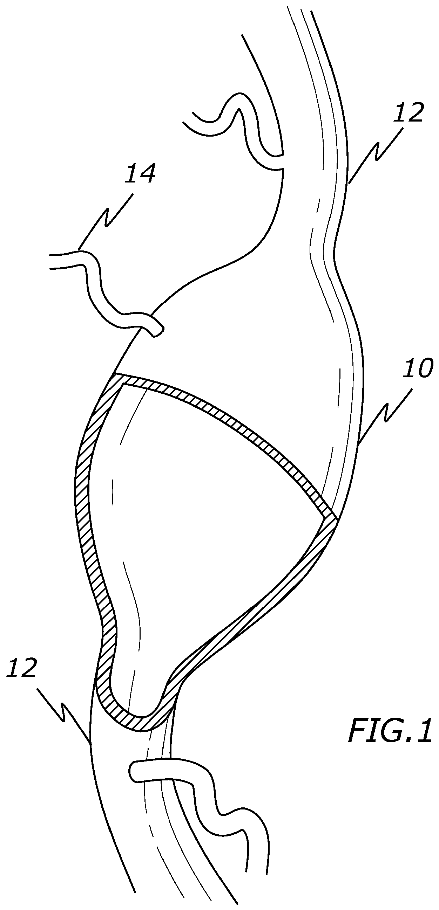

Aneurysms that expand on most or all sides of a vessel typically are known as fusiform aneurysms and represent about 3-13% of all intracranial aneurysms. Often, these fusiform aneurysms are found in the middle cerebral artery (MCA), the internal carotid artery (ICA), and the anterior cerebral artery (ACA). An example fusiform aneurysm 10 can be seen in Fig. 1 in which an area between adjacent vessel regions 12 bulge radially outward to form the fusiform aneurysm 10. Depending on the location of the fusiform aneurysm 10, it may be connected with additional, smaller vessels 14 that may feed other areas of the patient.

Due to their size and bulging on multiple sides along a vessel wall, fusiform aneurysm treatment can be challenging. Conventional treatment techniques include the use of a flow-diversion stent to divert flow away from the bulging side regions and to form an endothelial layer along the aneurysm neck over time. Since these aneurysms can be relatively large, the flow diversion stent may not always be long enough to effectively extend across the entire treatment region with enough overlap or radial force to stay in place.

Stent assisted coiling can also be used, whereby a stent is placed across the vessel while coils are separately introduced into the various sections of the fusiform aneurysm. However, this technique also presents challenges since the coils are introduced into multiple sides of the bulging vessel section.

In paten publication number US 2019/0269533 A1, a stent and stent delivery device are disclosed.

What is needed is a fusiform aneurysm treatment that better addresses the shortcomings of the current treatment techniques.

### SUMMARY OF THE INVENTION

The present invention is defined by the appended claims only, in particular by the scope of appended independent claims. References to "embodiments" throughout the description which are not under the scope of the appended claims merely represents possible exemplary executions and are therefore not part of the present invention.

This specification is generally directed to stents that are configured to better deploy and remain implanted across a fusiform aneurysm. Specifically, these stents include one or more anchoring members that radially expand within a fusiform aneurysm. In some instances, the anchoring members radially expand to a diameter that is larger than that of regions of vessels that are adjacent to the fusiform aneurysm.

In some embodiments, the anchoring mechanism may comprise an outer stent layer that radially expands to a bulbous shape. The largest expanded diameter of the bulbous shape may be larger than the diameter of regions of vessel that are adjacent to fusiform aneurysm, thereby preventing the stent from migrating out of the fusiform aneurysm. The outer stent layer may be disposed over a tubular flow diverting layer that creates a tubular passage through the fusiform aneurysm similar in size to the regions of vessel adjacent to the fusiform aneurysm.

In some embodiments, the anchoring mechanism may be a plurality of radially expandable structures, such as loops, arms, longitudinal wires, and/or hydrogel rings. These structures can be fixed on the outside of a generally cylindrical tubular braided stent with one or two layers. The radially expandable structures can be located at or near the proximal and distal ends, as well as any locations in between.

### BRIEF DESCRIPTION OF THE DRAWINGS

These and other aspects, features and advantages of which embodiments of the invention are capable of will be apparent and elucidated from the following description of embodiments of the present invention, reference being made to the accompanying drawings, in which:
Fig. 1 is a view of a fusiform aneurysm.
Fig. 2 is a view of a stent deployed within a fusiform aneurysm according to the present disclosure.
Fig. 3 is a view of the stent of Fig. 2 according to the present disclosure.
Figs. 4A, 4B, 4C, and 4D are views of the stent of Fig. 2 according to the present disclosure.
Fig. 5 is a view of a dual layer stent according to the present disclosure.
Fig. 6 is a magnified view of one end of the stent of Fig. 5 according to the present disclosure.
Fig. 7 is a view of the stent of Fig. 5 within a fusiform aneurysm according to the present disclosure.
Fig. 8 is a view of the inner flow diverting layer of the stent of Fig. 5 according to the present disclosure.
Fig. 9 is a view of the outer anchoring layer of the stent of Fig. 5 according to the present disclosure.
Fig. 10 is a view of a single layer stent according to the present disclosure.
Fig. 11 is a view of the stent of Fig. 10 within a fusiform aneurysm according to the present disclosure.
Fig. 12 is a view of one end of the stent of Fig. 10 according to the present disclosure.
Fig. 13 is a view of a single layer stent according to the present disclosure.
Fig. 14 is a view of the stent of Fig. 13 within a fusiform aneurysm according to the present disclosure.
Fig. 15 is a view of a stent with a plurality of arms according to the present disclosure.
Fig. 16 is a view of a stent with hydrogel rings according to the present disclosure.
Fig. 17 is a view of a stent with a plurality of longitudinal curved wires according to the present disclosure.
Figs. 18-21 illustrate a stent.

### DETAILED DESCRIPTION

Specific embodiments of the invention will now be described with reference to the accompanying drawings. This invention may, however, be embodied in many different forms and should not be construed as limited to the embodiments set forth herein; rather, these embodiments are provided so that this disclosure will be thorough and complete, and will fully convey the scope of the invention to those skilled in the art. The terminology used in the detailed description of the embodiments illustrated in the accompanying drawings is not intended to be limiting of the invention. In the drawings, like numbers refer to like elements.

As previously discussed, a fusiform aneurysm 10 refers to an aneurysm that expands on most or all sides of a vessel relative to the diameters of upstream and downstream vessel regions 12, as seen in Fig. 1. It is generally desirable to restore a passage through the fusiform aneurysm 10 that has a similar diameter to that of the vessel regions 12 on either side of the aneurysm 10 so as to prevent any blood flow pressures within the area of the aneurysm 10 that might further increase its diameter. In some cases, the area of the aneurysm 10 may be connected to smaller vessels 14 that feed other areas, and therefore it may sometimes be desirable to reinforce a fusiform aneurysm, reducing the flow against the walls of the aneurysm 10, but not completely closing off all blood flow to the feeder vessels 14. However, if no feeder vessels 14 are present or are supplying blood to a less important area, it may be desirable to substantially block all blood flow to the walls of the aneurysm 10.

This specification includes embodiments of stents that are configured to better deploy and remain implanted across a fusiform aneurysm 10. Deployment and retention can be improved in several different ways, including the use of one or more radially expandable structures such as an outer anchoring layer that conforms to the fusiform aneurysm, or a relatively long stent with loops, arms, longitudinal wires, or hydrogel rings at each end that better engages the interior of the aneurysm 10 and prevent the stent from migrating. In some embodiments, stents may include only one type of radially expandable structures or may alternately include two or more types of radially expandable structures.

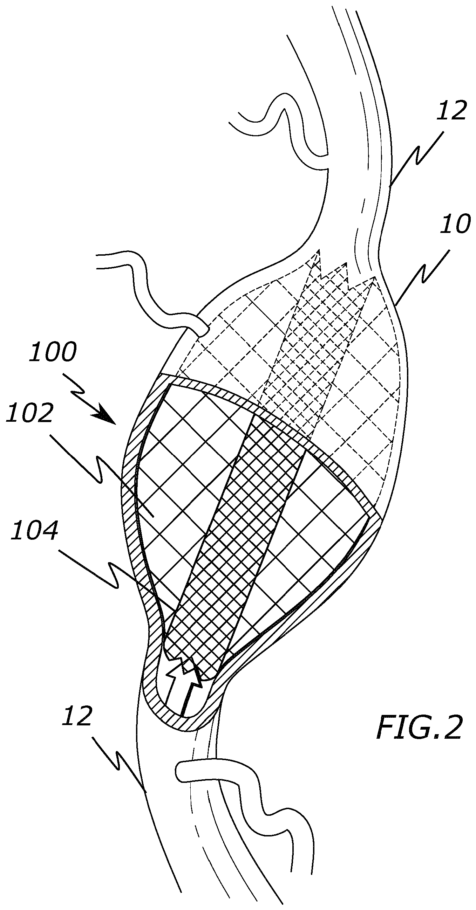

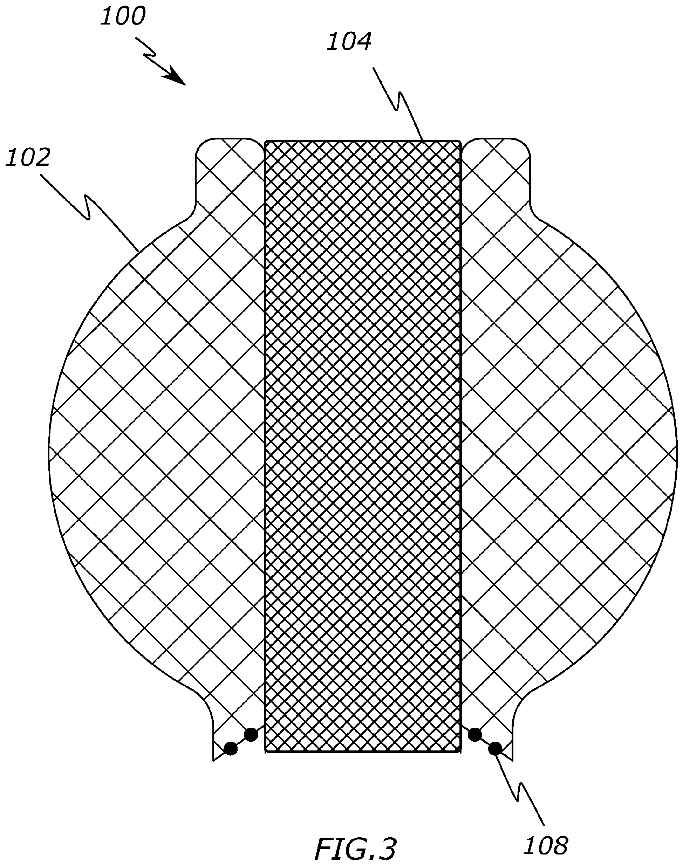

Figs. 2 and 3 illustrate one embodiment of an aneurysm treatment stent 100 for a fusiform aneurysm 10. More specifically, the stent 100 may include an outer anchoring layer 102 with an expanded shape configured to conform to the fusiform aneurysm 10, and an inner layer 104, relative to the outer anchoring layer 102, that has a similar diameter to the adjacent regions of vessels 12. The outer anchoring layer 102 expands against and helps anchor within the fusiform aneurysm 10 while the inner layer 104 forms a passage that may be generally continuous between each section of vessels 12.

The stent 100 may generally have a radially compressed configuration that allows delivery via a delivery catheter, as well as a radially expanded configuration seen in Figs. 2 and 3. This allows the stent 100 to be delivered within a fusiform aneurysm 10 so that the outer anchoring layer 102 expands against and conforms to the wall of the aneurysm 10 and so that the inner layer 104 forms a cylindrical or tubular passage through the outer anchoring layer 102 to bridge the gap between vessels regions 12. Fig. 3 best illustrates the stent 100 alone while Fig. 2 illustrates the stent 100 delivered within the fusiform aneurysm 10.

In one example, the outer anchoring layer 102 and the inner layer 104 may be both composed of a woven or braided wire mesh. In other words, one or more wires are braided together to form both the outer anchoring layer 102 and the inner layer 104. The entire device may be woven from the same wires and/or wires of the same diameter. Alternately, the outer anchoring layer 102 and the inner layer 104 may be woven from different diameter wires (i.e., the wires of the outer anchoring layer 102 may be larger in diameter than those of the inner layer 104). Additionally, each layer 102, 104 may have several different diameter wires that make up each layer.

At least some of the wires of the stent 100 may preferably be composed of a shape memory material, such as Nitinol or similar alloy that allows an expanded secondary shape to be imparted to it.

In one specific example, the wire of the outer anchoring layer 102 has a diameter within an inclusive range of about 0.0254 mm, i.e. about 0.001 inch and 2.54 mm, i.e. 0.10 inch in diameter, and more particularly within an inclusive range of about 0.04572 mm, i.e. about 0.0018 inch and about 0.127 mm, i.e. about 0.0050 inch. The inner layer 104 may be composed of wires having a diameter within an inclusive range of abou t0.0127 mm, i.e. about 0.0005 inch and about 0.04572 mm, i.e. about 0.0018 inch.

The porosity of the outer anchoring layer 102 may be generally more porous (i.e., have larger sized pores) than the inner layer 104. This may allow the outer anchoring layer 102 to better exert anchoring force while allowing the inner layer to better divert or block blood flow through its walls. In one example, the outer anchoring layer 102 may have a porosity within an inclusive range of about 75% to 95%, and more preferably an inclusive range between about 80% to 88%. The inner layer 104 may have a porosity within an inclusive range of about 45 to 70%.

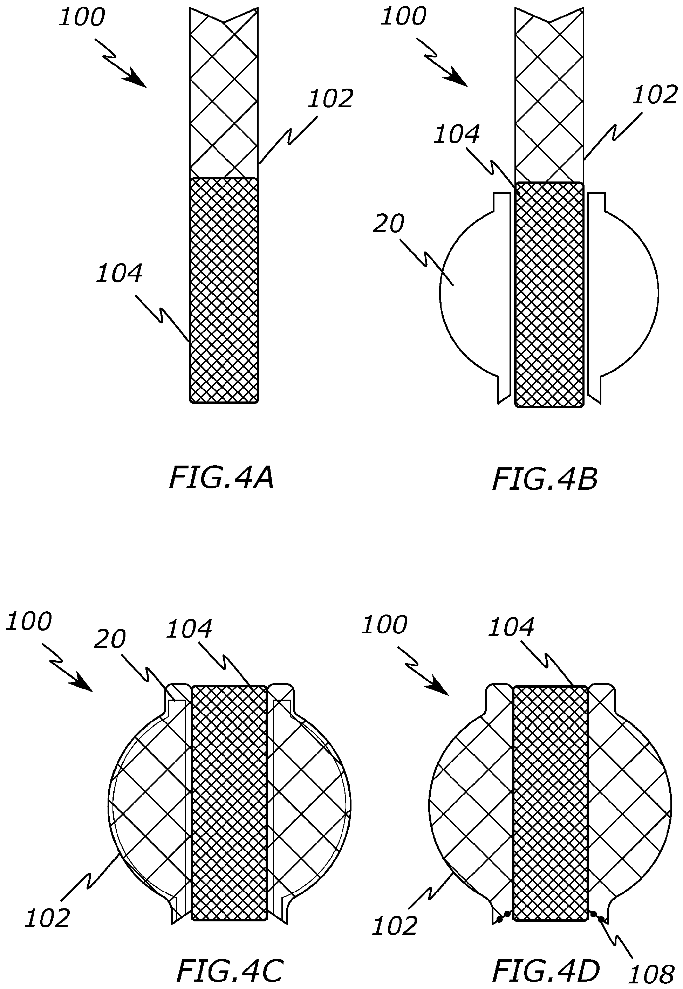

The stent 100 can be created in several different ways, one of which is shown in Figs. 4A-4D. First, an initial tube may be created with the ultimate outer anchoring layer 102 and inner layer 104. This tube can be created by weaving two different tubular structures with similar diameters separately and then attaching them together via welding, wire loops, coils, marker bands, or similar attachment mechanisms. Alternately, this initial tube can be created by using a single mandrel and braiding wires on the mandrel in two different patterns along its length. For example, only a single wire can be braided longitudinally or a plurality of wires can be braided.

Once the initial tubular structure is created, it can be placed over a mandrel 20, as seen in Figs. 4B and 4C. The mandrel can be generally shaped similar to the desired expanded shape of the stent 100, including an inner tubular portion and an outer bulbous region. In that respect, the portion that is to become the inner layer 104 can be placed within the passage or tubular portion of the mandrel 20, as seen in Fig. 4B. Next, the region that is to become the outer anchoring layer 102 can be bent over the outside of the bulbous region of the mandrel 20.

Depending on how the stent 100 is deployed, the stent 100 can then be heat set to the desired shape of the mandrel 20 or can be both heat set and its free ends connected at its proximal end at areas 108. More specifically, the stent 100 may be compressed within a delivery catheter in the shape shown in Fig. 3 and therefore always maintains a generally larger or smaller size and layer configuration of its ultimate shape. Or alternately, the stent 100 may be compressed within a delivery catheter in the single layer tubular configuration of Fig. 4A and folded back on itself during delivery.

In the first instance, it may be desirable that either before or after heat setting that the bottom edges of the tubular structure be connected to each other via connecting members. These connecting members can be wire (e.g., woven circumferentially through both layers), coils, welded areas, or similar connection mechanisms. Additionally, any of these connecting mechanisms may include or be composed of radiopaque material.

In the second instance, both ends of the initial tubular structure are not connected together, but the heat set shape resembles that of Fig. 3. This allows the outer anchoring layer 102 to first be deployed along most or all of the length within the fusiform aneurysm 10, then inverted or folded inside out so that the inner layer 104 is deployed in a tubular shape within the outer anchoring layer 102.

In either instance, the stent 100 forms a tubular shape with the inner layer 104 that is similar to the two adjacent vessel regions 12 and further creates a separate, enclosed bulbous anchoring portion from the outer anchoring layer 102.

While not shown in the figures, the proximal and/or distal ends of the stent 100 may include anchoring members. These anchoring members may take the form of a plurality of radially expandable loops, a plurality of wire coils on regions of the wire at each end, barbs, spikes, or similar engagement mechanisms. In one specific example, the anchoring members may include a plurality of wire loops that have coils on the wire of one or more of the loops.

One or more areas of the stent 100 may also include a hydrogel coating. For example, the inner layer 104 may include a hydrogel coating that gradually expands in size once deployed within a patient.

While the stent 100 at least partially relies on the bulbous shape of its outer anchoring layer 102, alternate stent embodiments may use other anchoring features to help retain the stent within the fusiform aneurysm 10. One such example is that the stent can include a plurality of radially extending structures that expand from an outer circumference of the stent in one or more areas. These radially extending structures preferably expand to a diameter larger than that of the diameter of the adjacent vessels 12 so as to help prevent the stent from migrating out of the fusiform aneurysm 10.

The radially extending features can have a variety of different forms. For example, these features may be a plurality of radially extending wire loops, wire triangular shapes, wire arms, wire hooks, longitudinally curved wires, or hydrogel rings that radially expand when exposed to blood.

The radially extending features are preferably located so that they expand within or close to the fusiform aneurysm. In this respect, the features can be located along the length of the stent at the very proximal and distal ends of the stent, offset towards a middle of the stent from each end (e.g., by 5%, 10%, 15%, 20% or more of the total stent length), near a middle of the stent, or any combination of these locations.

The radially extending features may expand to a radial size that is larger than the radius of the stent body. The exact size may vary based on the size of the stent and the size of the fusiform aneurysm 10. However, the radially extending features may expand to a diameter relative to the stent body that is 5%, 10%, 15%, 20%, 25%, 30%, 35%, 40%, or amounts in between these values.

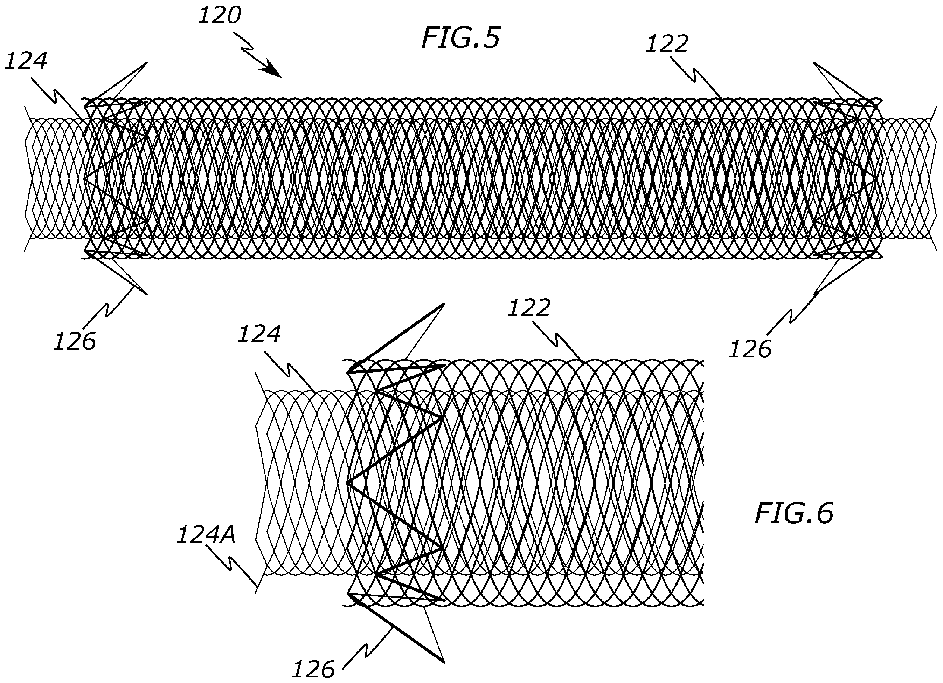

One specific embodiment of a stent 120 that includes a plurality of radially extending features is illustrated in Figs. 5-7. Specifically, the stent 120 includes a plurality of loops 126 that are configured to radially expand or flare away from the body of the stent 120.

The stent 120 may be composed of an outer anchoring layer 122 and an inner flow diverting layer 124 that is located within an inner passage of the outer anchoring layer 122. The two layers 122, 124 can be attached to each other by braiding one or more connecting wires between the two layers 122, 124, by using one or more connecting members (e.g., a wire loop, coil, or band), or by welding.

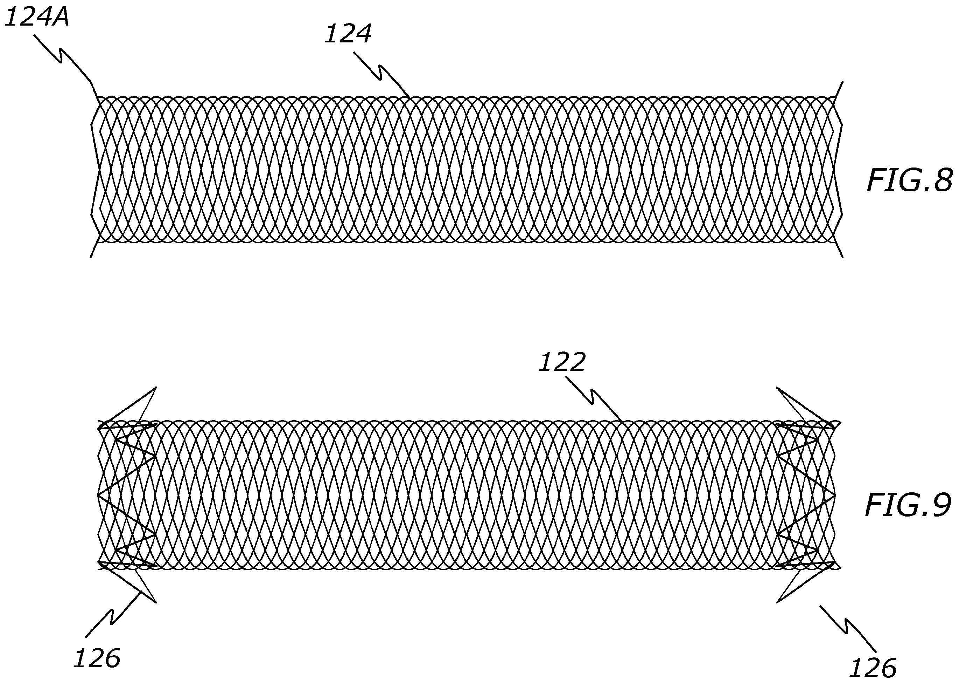

The two individual layers 122, 124 can be separately seen in Figs. 8 and 9. The outer anchoring layer 122 may generally have a higher porosity and larger wire size than that of the inner flow diverting layer 124, such that the outer anchoring layer 122 can better anchor the stent 120 and the inner flow diverting layer 124 can prevent blood flow from passing into the fusiform aneurysm 10. These outer and inner layers 122, 124 may have similar example characteristics (e.g., wire size and porosity) as the previously described layers 102 and 104, respectively.

Since it may be desirable to expand the plurality of loops 126 within the fusiform aneurysm 10, it may also be desirable that the inner flow diverting layer 124 extends proximally and distally beyond the ends of the outer anchoring layer 122, as best seen in the magnified view of Fig. 6. For example, the inner flow diverting layer 124 may extend beyond the outer anchoring layer 122 by 5%, 10%, 15%, 20%, 25%, or any percent in between, relative to the length of the outer anchoring layer 122. The proximal and distal ends of the inner flow diverting layer 124 may also include a plurality of relatively smaller flared loops 124A that may help engage the walls of the adjacent vessels 12. Alternately, the inner flow diverting layer 124 may have proximal and distal ends that terminate at about the proximal and distal ends of the outer anchoring layer 122.

The loops 126 can be composed of wire. This wire can be either separately attached to the braided tubular body of the outer anchoring layer 122 or can be formed during the braiding process with the wire that also forms the braided tubular body of the outer anchoring layer 122. If separately attached, a ring forming a plurality of loops 126 can be formed and then attached via welding, wire loops, wire coils, or similar attachment mechanisms.

The loops 126 may all be about the same size or may alternate between larger and smaller loops. The loops 126 may be heat set to radially expand or flare outwards from the main tubular body of the outer anchoring layer 122. This can be achieved by expanding outward at an angle relative to a longitudinal axis of the body of the stent 120 within an inclusive range of 5 degrees to 90 degrees towards a middle of the stent 120. The loops may each form a relatively flat plane or can be configured to gently curve or arc outwards away from the body of the stent 120.

The size of the loops 126 may vary, depending on the heat set angle that the loops 126 expand away from the stent 120 and the desired circumferential size the loops are to expand to. Again, the expanded circumferential size of the loops 126 may expand to a diameter relative to that of the stent body that is 5%, 10%, 15%, 20%, 25%, 30%, 35%, 40% larger, or amounts in between these values.

The loops 126 are depicted as having a sharp, triangular shape. However, other shapes are possible, such as a rounded or circular shape. While described as loops, the loops 126 might alternately be a circular wire that repeat wave shapes that are fixed to the stent.

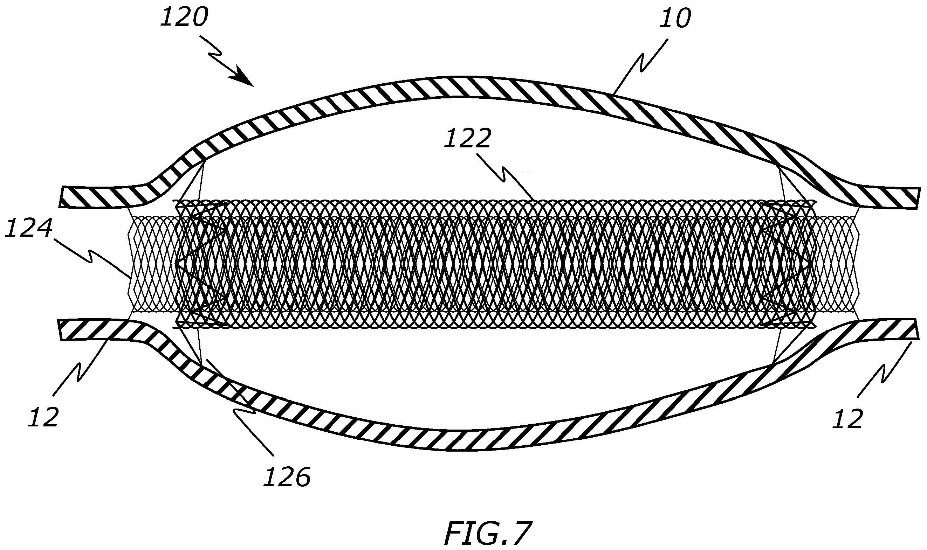

In the present example stent 120, the loops 126 are connected at a very distal end or edge and proximal end or edge of the outer anchoring layer 122. Since the inner flow diverting layer 124 further extends proximally and distally, the stent loops 126 are positioned somewhat away from the ends of the stent 120 as a whole, allowing the loops 126 to expand within the fusiform aneurysm 10 and allowing the inner flow diverting layer 124 to engage the smaller diameter adjacent vessels 12, as seen in Fig. 7. This allows the flow diverting layer 124 to creating a continuous passage through the fusiform aneurysm 10 while also allowing the outer anchoring layer to better anchor within the aneurysm 10 and prevent stent migration.

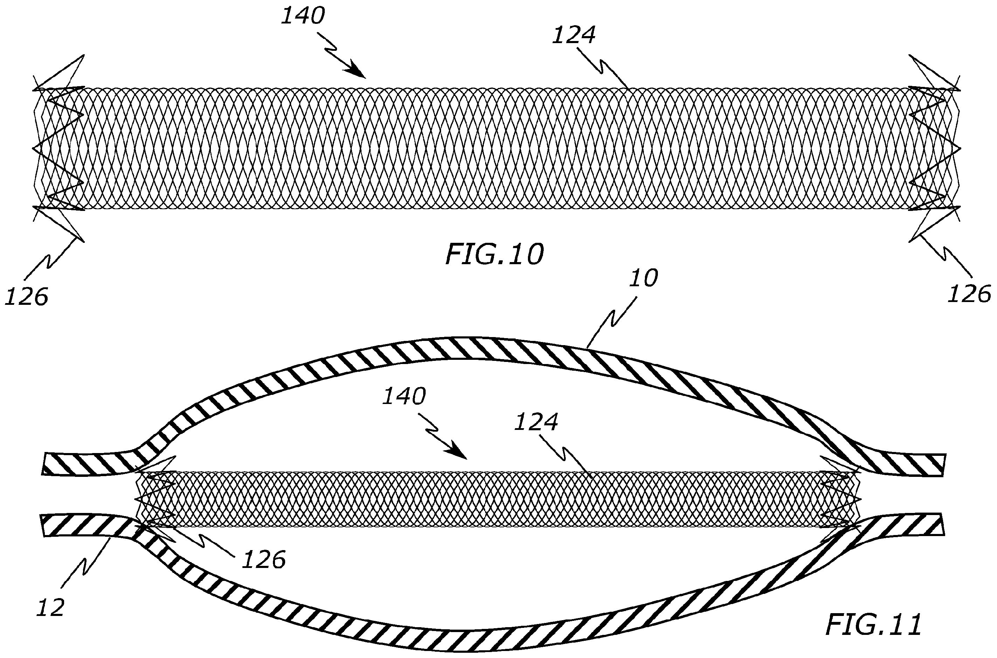

Alternately, only a single stent layer may be used. For example, Figs. 10 and 11, illustrate a stent 140 composed of only a flow diverting layer 124. The loops 126 (or radially extending features) may be connected directly to the flow diverting layer 124 in a similar manner and size as previously discussed. Alternately, only the outer anchoring layer 122 can be used, though if the porosity is not sufficient to block blood into the aneurysm 10, the layer 122 may include an expandable hydrogel coating, polymer liner or similar material.

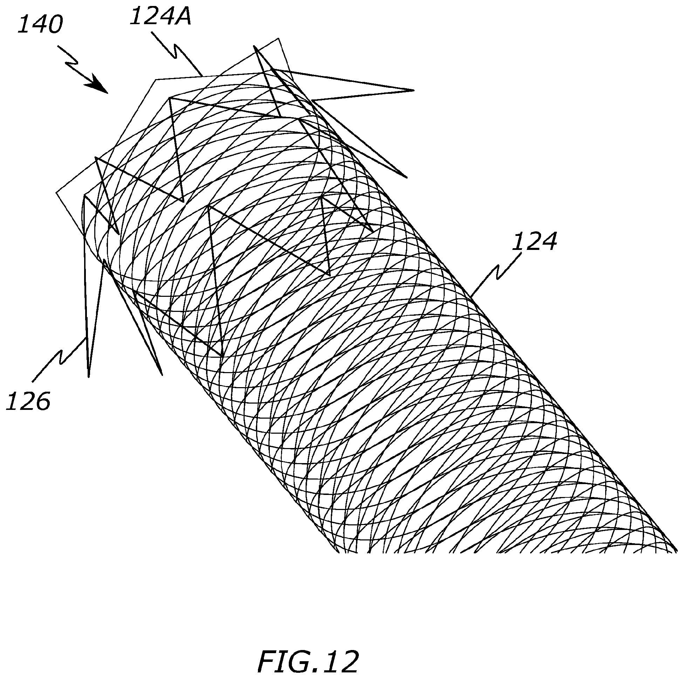

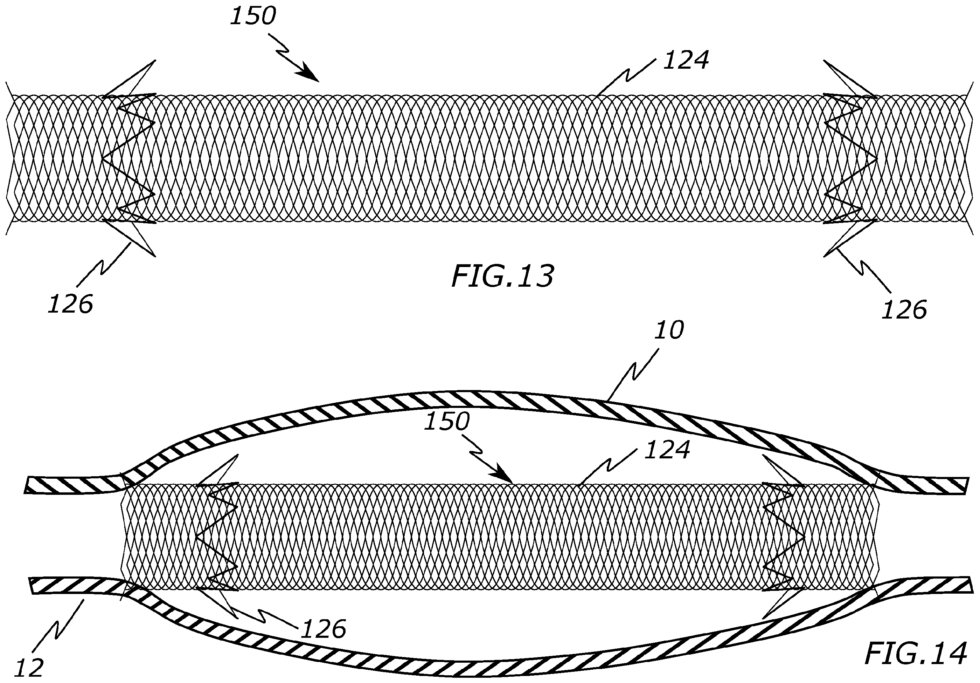

In the present example stent 140, the loops 126 are positioned at both the very proximal and distal ends of the flow diverting layer 122, as best seen in Fig. 12. However, the loops 126 may also be positioned away from the proximal and distal ends, as seen with stent 150 in Figs. 13 and 14. In that respect, the loops 126 may be located at longitudinal positions of the total length of either stent of 0%, 5%, 10%, 15%, 20%, and percentages in between.

Any of the stents 120, 140, and 150 may additionally have loops 126 located near the middle of the stent. Optionally these middle-positioned loops 126 may have a larger diameter than those closer to the proximal and distal ends of the stent.

In another example, any of the stents 120, 140, and 150 may have multiple circumferential rings of loops 126. For example, the stents may have 2, 3, 4, 5, 6, 7, 8, 9, 10, or more. These rings of loops 126 may be positioned at equal longitudinal positions from each other or may be positioned at different distances. The rings of loops 126 may also increase or decrease in their expanded radial diameter relative to adjacent rings of loops 126. For example, the rings of loops may increase in diameter towards the middle of the stent or may alternate between larger and smaller diameter rings of loops 126.

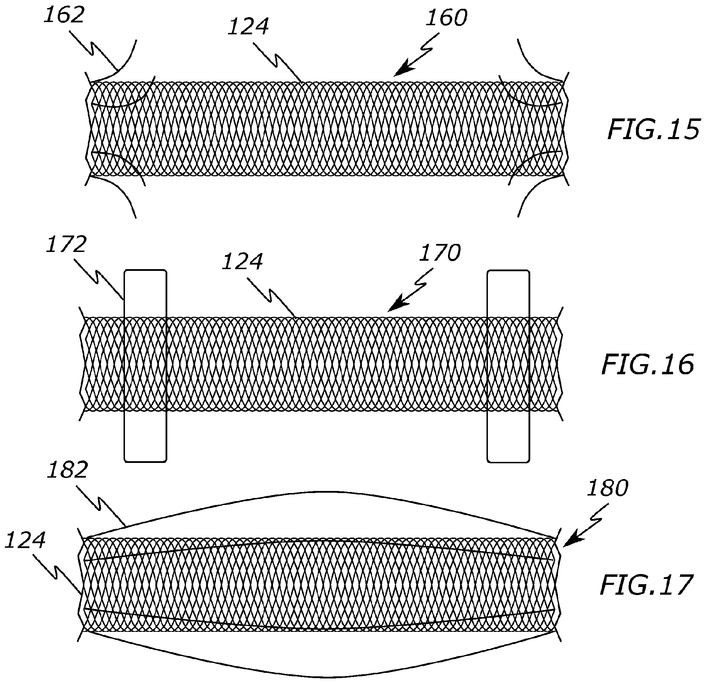

Again, while loops 126 are specifically shown, other radially expandable structures may alternately be used in its place. For example, a plurality of wire arms 162 may bend radially outward from the stent 160, as seen in Fig. 15. The ends of the arms 162 may include blunt ends or hooks.

In another example seen in Fig. 16, hydrogel rings 172 can be attached to the stent 170 and can radially expand after delivery. The hydrogel rings 172 may have a relatively thin profile for delivery but may greatly radially expand once delivered within the patient and exposed to blood.

In another example seen in Fig. 17 includes a plurality of longitudinal wires 182 that are fixed near the proximal and distal ends of the stent 180. The wires 182 may radially expand to an arc shape that has a larger diameter than adjacent vessels 12. The stent 180 may include 2, 3, 4, 5, 6, 7, 8, or more wires 182 connected around an outer circumference of the stent 180. Alternately, the wires 182 may be fixed at shorter distances than nearly the entire length of the stent 180, such as between a proximal/distal end and a middle of the stent 180. Hence, the stent 180 may have separate sets of wires on its proximal half and its distal half.

Any of the radially expandable structures may be configured such that they expand at an angle between 5 degrees to 90 degrees towards a middle of the stent and relative to a longitudinal axis of a stent. In other words, their radial position/size increases towards a middle of the stent. In some instances where the radially expandable structures are positioned away from the very distal and proximal ends, it may be desirable to configure the radially expandable structures to expand in an opposite angle, namely, between 5 degrees to 90 degrees away from a middle of the stent and relative to a longitudinal axis of a stent.

Alternately, a combination of loops 126, arms 162, wires 182, and/or hydrogel rings 172 may be used at various longitudinal positions along the stent.

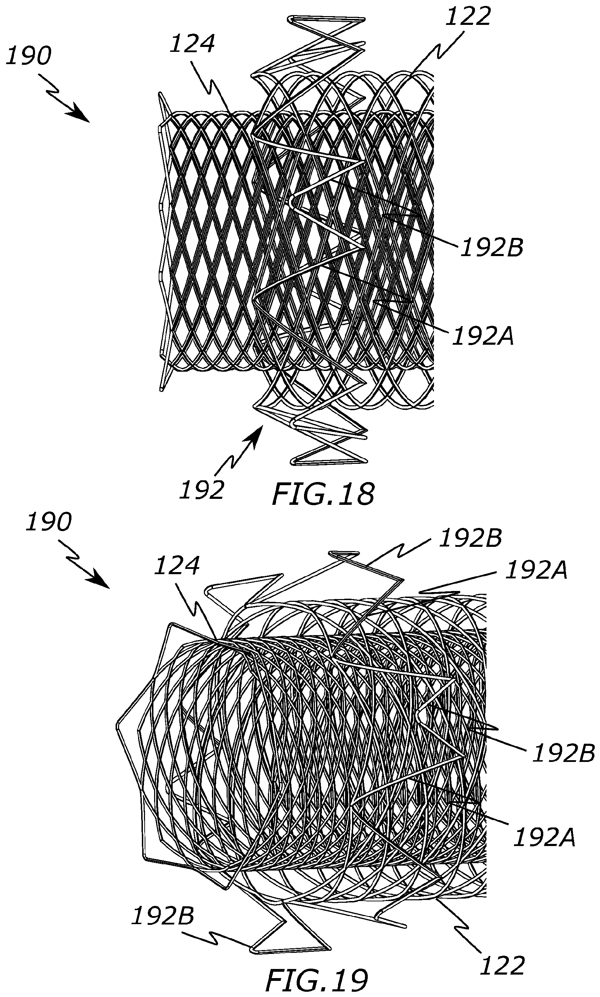

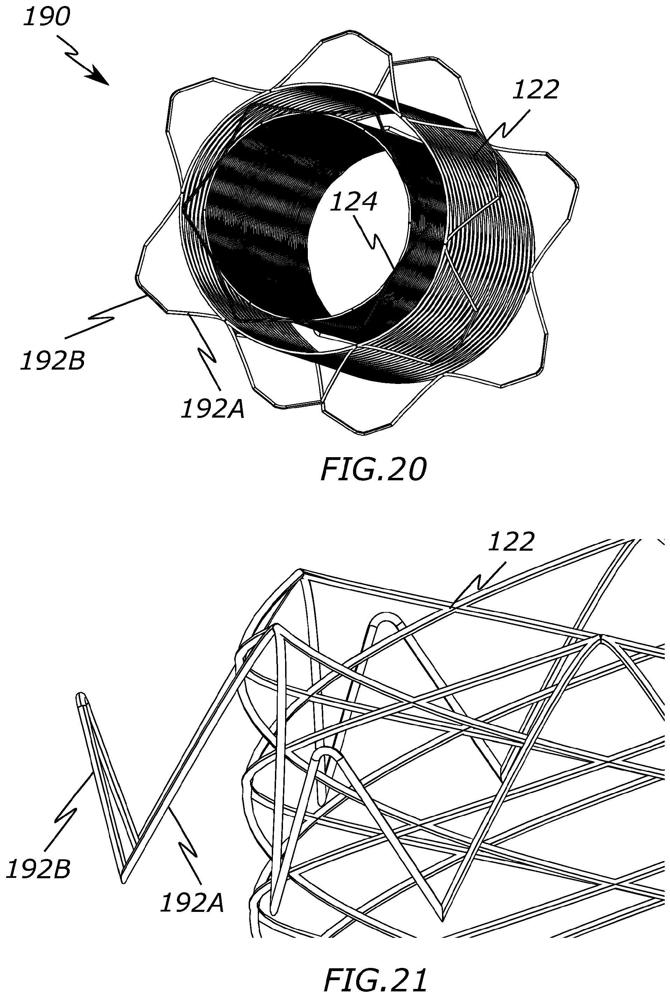

The loops 126 have been previously described and depicted as having a rounded or triangular/pointed shape. However, more complicated loop shapes are also possible. For example, Figs. 18-21 illustrate a stent 190 that is generally similar to stent 120 but has a plurality of loops 192 that bend backward on themselves. In other words, part of the loop extends at a first angle and another part of the loop extends at a different angle. This shape creates wire peaks in both proximal and distal direction which may help anchoring the stent 190.

Specifically, each loop has a wider portion 192A that extends from and is fixed near an edge of the anchoring layer 122. This wider portion 192A extends at an angle both towards a middle of the anchoring layer 122 and radially outward from the anchoring layer 122. A narrower portion 192B that forms a loop end or tip may be folded back relative to the wider portion 192A so that it extends back towards the edge of the anchoring layer 122 and further radially outward from the anchoring layer 122.

Another way to describe this feature is that each of the loops 192 form a peak angled towards a middle of the stent 190, followed by a peak angled away from the middle of the stent 190, followed by a peak angled towards the middle of the stent 190. Further, the middle peak may be radially further away from the stent 190 than the two side peaks.

The fold or inflection point between the widest portion 192A and narrower portion 192B can be at almost any position along the length of the loop 192. For example, the inflection point may occur at 20%, 30%, 40%, 50%, 60%, 70%, or 80% of the length of the loop 192, as well as location in between these values. All of the loops 192 are depicted as being the same size and have inflection points between the portions 192A, 192B at the same relative location. However, these loops 192 may have different sizes, such as alternating between larger and smaller loops. Additionally or alternately, the loops 192 may have different inflection point locations, such as alternating between different inflection points (e.g., between a location at 60% and 40% of loop length).

Relative to the longitudinal axis of the stent 190, the wider portion 192A and narrow portion 192B can form a variety of different angles. For example, the wider portion 192A may form an angle of 10, 20, 30, 40, 50, 60, 70, 80, or 90 degrees, as well as angles in between these values. In another example, the narrower portion 192B may form an angle of 90, 100, 110, 120, 130, 140, 150, 160, 170, or 180, as well as angles in between these values. Again, these angles are both relative to the longitudinal axis of the stent 190.

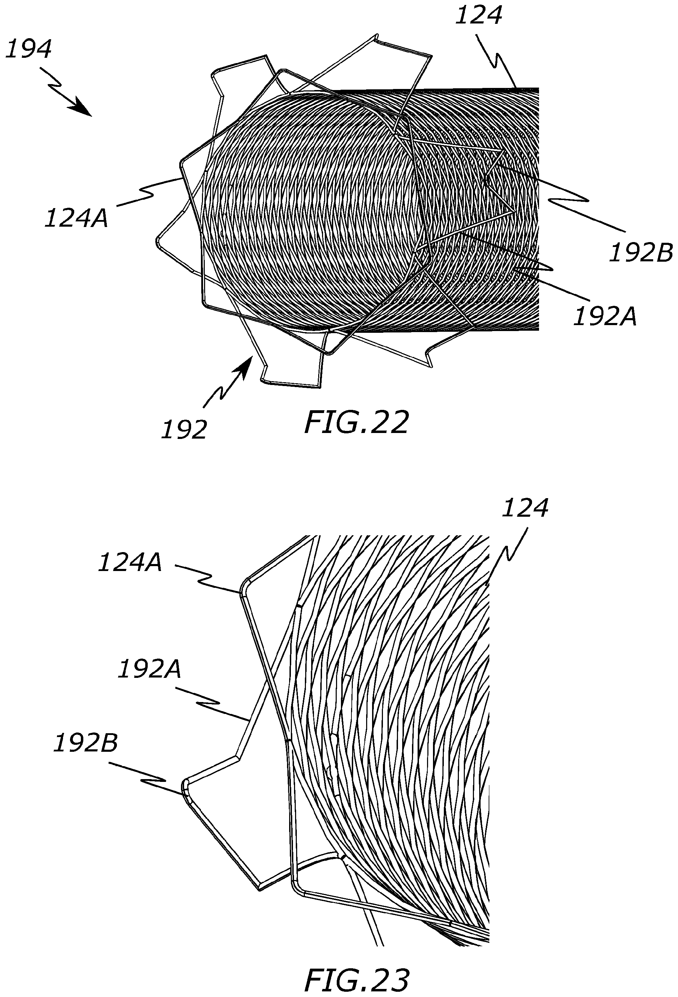

These loops 192 with two different bend angles can also be used on a single layer stent 194, which is similar to the previously described stent 140. In this example, the stent 194 includes both loops 192 and the smaller loops 124A that form a rounded or triangular loop shape away from the body of the stent 140. The loops 124A can be positioned and fixed to the flow diverting layer 124 so that each loop 124A partially overlaps two adjacent loops 192. These loops 124A can fixed above the loops 192 (i.e., on the side opposite the stent body as seen in Fig. 23) or can be fixed below loops 192 (i.e., on the same side as the stent body) to help outwardly bias the larger loops 192.

Any of the previously described stents can generally be deployed by deploying a distal end of the stent within or near a first adjacent portion of a vessel 12, deploying a middle section of stent along an interior of a fusiform aneurysm 10, and finally deploying a proximal end of the stent within or near a second adjacent portion of a vessel 12. This method may also include expanding anchoring elements such as an outer anchoring layer or radially expandable structure.

The previously described stent 100 may additionally include the radially expandable structures. These may by positioned, for example, near the proximal and distal ends of the stent 100 so that they do not interfere with expansion of the outer anchoring layer 102.

The stents of this specification may have a variety of different sizes and diameters, depending on their location of use. Generally, all of the stents in this specification may have an example length within an inclusive range of 12mm to 35mm (e.g., 12mm, 14mm, 16mm, 18mm, 20mm, 22mm, 24mm, 26mm, 28mm, 30mm, 32mm, and 34mm. Generally, the dual layer stents of this specification (e.g., stent 120 and 190) have an inner lumen diameter of the flow diverting layer 124 within an inclusive range of 2.5mm to 5mm (e.g., 2.5mm, 3mm, 3.5mm, 4mm, 4.5mm, and 5mm). Generally, the dual layer stents of this specification (e.g., stent 120 and 190) have an inner lumen diameter of the anchoring layer 122 within an inclusive range of 2.8mm to 5.5mm (e.g., 2.8mm, 3mm, 3.5mm, 4mm, 4.5mm, 5mm, and 5.5mm). Generally, the single layer stents of this specification (e.g., stent 140 and 194) have an inner lumen diameter of the flow diverting layer 124 within an inclusive range of 2.5mm to 5.5mm (e.g., 2.5mm, 3mm, 3.5mm, 4mm, 4.5mm, 5mm, and 5.5mm).

While different elements or features of a stent are shown in each embodiment, it is specifically contemplated that any of these features described herein can be mixed, matched, and otherwise combined with each other.

Although the invention has been described in terms of particular embodiments and applications, one of ordinary skill in the art, in light of this teaching, can generate additional embodiments and modifications without departing from or exceeding the scope of the claimed invention. Accordingly, it is to be understood that the drawings and descriptions herein are proffered by way of example to facilitate comprehension of the invention and should not be construed to limit the scope thereof.

## Claims

1. A stent (190) for deploying across a fusiform aneurysm, comprising;
a stent body (124) having a tubular expanded shape; and
a radially expandable structure (122) positioned on an outer surface of the stent body; the radially expandable structure having an expanded diameter larger than the tubular expanded shape of the stent body;
**characterized in that** the radially expandable structure comprises one or more rings formed from a plurality of radially expanded wire loops (192); wherein each of the one or more rings include a first plurality of peaks pointing in a proximal direction and a second plurality of peaks pointing in a distal direction; and wherein the first plurality of peaks and the second plurality of peaks each define a free end.

2. The stent of claim 1, wherein the one or more rings comprises a first ring connected at a distal end of the stent body and a second ring connected at a proximal end of the stent body.

3. The stent of claim 2, wherein the first ring is connected spaced away from a distal edge of the stent body and wherein the second ring is connected spaced away from a proximal edge of the stent body.

4. The stent of claim 2, further comprising a third ring connected near a middle of the stent body.

5. The stent of claim 1, wherein the one or more rings comprises a plurality of rings connected along a length of the stent body.

6. The stent of claim 1, wherein the plurality of radially expanded wire loops are angled within an inclusive range of 5 to 90 degrees towards a middle of the stent and relative to a longitudinal axis of the stent.

7. The stent of claim 1, wherein the plurality of radially expanded wire loops are angled within an inclusive range of 5 to 90 degrees away from a middle of the stent and relative to a longitudinal axis of the stent.

8. The stent of claim 1, wherein each of the plurality of radially expanded wire loops form a flat plane.

9. The stent of claim 1, wherein each of the plurality of radially expanded wire loops form an arc shape curving radially outwards from the stent body.

10. The stent of claim 1, wherein the plurality of radially expanded wire loops have a diameter that is 5%, 10%, 15%, 20%, 25%, 30%, 35%, 40% larger than a diameter of the stent body.

11. The stent of claim 1, wherein the plurality of radially expanded wire loops comprise hydrogel.

12. The stent of claim 1, wherein the plurality of radially expanded wire loops each have a first part of the loop angled at a first angle relative to a longitudinal axis of the stent, and a second part of the loop angled at a second angle relative to the longitudinal axis of the stent.

13. The stent of claim 12, wherein the first part of the loop is wider than the second part of the loop.

14. The stent of claim 13, wherein the first part of the loop extends towards a middle of the stent and wherein the second part of the loops extends away from the middle of the stent.

## Patentansprüche

1. Ein Stent (190) zur Behandlung eines fusiformen Aneurysma, der folgendes umfasst:
einen Stentkörper (124) mit einer röhrenförmig expandierten Form; und
eine radial expandierbare Struktur (122) an einer Außenfläche des Stentkörpers; wobei die radial expandierbare Struktur einen expandierten Durchmesser aufweist, der größer ist als die röhrenförmig expandierte Form des Stentkörpers;
**dadurch gekennzeichnet, dass** die radial expandierbare Struktur einen oder mehrere Ringe umfasst, die aus einer Vielzahl von radial expandierten Drahtschlaufen (192) gebildet sind; wobei jeder der einen oder mehreren Ringe eine erste Vielzahl von in proximaler Richtung weisenden Spitzen und eine zweite Vielzahl von in distaler Richtung weisenden Spitzen aufweist; und wobei die erste Vielzahl von Spitzen und die zweite Vielzahl von Spitzen jeweils ein freies Ende definieren.

2. Der Stent nach Anspruch 1, wobei der eine oder die mehreren Ringe einen ersten Ring, der an einem distalen Ende des Stentkörpers angebracht ist, und einen zweiten Ring, der an einem proximalen Ende des Stentkörpers angebracht ist, umfassen.

3. Der Stent nach Anspruch 2, wobei der erste Ring in einem Abstand von einer distalen Kante des Stentkörpers angebracht ist und wobei der zweite Ring in einem Abstand von einer proximalen Kante des Stentkörpers angebracht ist.

4. Der Stent nach Anspruch 2, der ferner einen dritten Ring umfasst, der nahe der Mitte des Stentkörpers angebracht ist.

5. Der Stent nach Anspruch 1, wobei der eine oder die mehreren Ringe eine Vielzahl von Ringen umfassen, die entlang einer Länge des Stentkörpers verbunden sind.

6. Der Stent nach Anspruch 1, wobei die mehreren radial expandierten Drahtschlaufen in einem Bereich von 5 bis 90 Grad in Richtung der Mitte des Stents und relativ zu einer Längsachse des Stents abgewinkelt sind.

7. Der Stent nach Anspruch 1, wobei die mehreren radial expandierten Drahtschlaufen in einem Bereich von 5 bis 90 Grad, einschließlich der Grenzwerte, von der Mitte des Stents weg und relativ zu einer Längsachse des Stents abgewinkelt sind.

8. Der Stent nach Anspruch 1, wobei jede der mehreren radial expandierten Drahtschlaufen eine ebene Fläche bildet.

9. Der Stent nach Anspruch 1, wobei jede der mehreren radial expandierten Drahtschlaufen eine Bogenform bildet, die sich radial vom Stentkörper nach außen wölbt.

10. Der Stent nach Anspruch 1, wobei die Vielzahl radial expandierter Drahtschlaufen einen Durchmesser aufweist, der um 5 %, 10 %, 15 %, 20 %, 25 %, 30 %, 35 % oder 40 % größer ist als der Durchmesser des Stentkörpers.

11. Der Stent nach Anspruch 1, wobei die mehreren radial expandierten Drahtschlaufen Hydrogel enthalten.

12. Der Stent nach Anspruch 1, wobei die mehreren radial expandierten jeweils einen ersten Teil der Schlaufe, der in einem ersten Winkel relativ zur Längsachse des Stents abgewinkelt ist, und einen zweiten Teil der Schlaufe, der in einem zweiten Winkel relativ zur Längsachse des Stents abgewinkelt ist, aufweisen.

13. Der Stent nach Anspruch 12, wobei der erste Teil der Schlaufe breiter ist als der zweite Teil der Schlaufe.

14. Der Stent nach Anspruch 13, wobei sich der erste Teil der Schlaufe in Richtung der Mitte des Stents erstreckt und wobei sich der zweite Teil der Schlaufen von der Mitte des Stents weg erstreckt.

## Revendications

1. Stent (190) destiné à être déployé à travers un anévrisme fusiforme, comprenant :
un corps de stent (124) présentant une forme déployée tubulaire ; et
une structure radialement déployable (122) positionnée sur une surface extérieure du corps de stent ; la structure radialement déployable présentant un diamètre déployé plus important que la forme déployée tubulaire du corps de stent ;
**caractérisé en ce que** la structure radialement déployable comprend une ou plusieurs bagues formées à partir d'une pluralité de boucles de fil radialement déployées (192), où chacune des une ou plusieurs bagues inclut une première pluralité de crêtes pointant dans une direction proximale et une seconde pluralité de crêtes pointant dans une direction distale ; et où la première pluralité de crêtes et la seconde pluralité de crêtes définissent chacune une extrémité libre.

2. Stent selon la revendication 1, dans lequel les une ou plusieurs bagues comprennent une première bague reliée au niveau d'une extrémité distale du corps de stent et une deuxième bague reliée au niveau d'une extrémité proximale du corps de stent.

3. Stent selon la revendication 2, dans lequel la première bague est reliée à l'écart d'un bord distal du corps de stent et où la deuxième bague est reliée à l'écart d'un bord proximal du corps de stent.

4. Stent selon la revendication 2, comprenant en outre une troisième bague reliée près d'un milieu du corps de stent.

5. Stent selon la revendication 1, dans lequel les une ou plusieurs bagues comprennent une pluralité de bagues reliées le long d'une longueur du corps de stent.

6. Stent selon la revendication 1, dans lequel la pluralité de boucles de fil radialement déployées sont inclinées dans les limites d'une plage inclusive allant de 5 à 90 degrés en direction d'un milieu du stent et par rapport à un axe longitudinal du stent.

7. Stent selon la revendication 1, dans lequel la pluralité de boucles de fil radialement déployées sont inclinées dans les limites d'une plage inclusive allant de 5 à 90 degrés en direction opposée d'un milieu du stent et par rapport à un axe longitudinal du stent.

8. Stent selon la revendication 1, dans lequel chacune de la pluralité de boucles de fil radialement déployées forme un plan plat.

9. Stent selon la revendication 1, dans lequel chacune de la pluralité de boucles de fil radialement déployées forme une forme d'arc s'incurvant radialement vers l'extérieur par rapport au corps de stent.

10. Stent selon la revendication 1, dans lequel la pluralité de boucles de fil radialement déployées présentent un diamètre qui est 5 %, 10 %, 15 %, 20 %, 25 %, 30 %, 35 %, 40 % plus important qu'un diamètre du corps de stent.

11. Stent selon la revendication 1, dans lequel la pluralité de boucles de fil radialement déployées comprennent un hydrogel.

12. Stent selon la revendication 1, dans lequel la pluralité de boucles de fil radialement déployées présentent chacune une première partie de la boucle inclinée selon un premier angle par rapport à un axe longitudinal du stent, et une seconde partie de la boucle inclinée selon un second angle par rapport à l'axe longitudinal du stent.

13. Stent selon la revendication 12, dans lequel la première partie de la boucle est plus large que la seconde partie de la boucle.

14. Stent selon la revendication 13, dans lequel la première partie de la boucle s'étend en direction d'un milieu du stent et où la seconde partie des boucles s'étend vers l'opposé du milieu du stent.
